(19) Europäisches Patentamt / European Patent Office / Office européen des brevets

(11) **EP 4 311 550 A1**

(12) **EUROPEAN PATENT APPLICATION**

(43) Date of publication:
**31.01.2024 Bulletin 2024/05**

(21) Application number: **22187606.3**

(22) Date of filing: **28.07.2022**

(51) International Patent Classification (IPC):
***A61K 31/409*** *(2006.01)* ***A61K 8/00*** *(2006.01)*
***A61K 36/00*** *(2006.01)* ***A61P 31/04*** *(2006.01)*
***C07D 487/00*** *(2006.01)* ***C12N 1/00*** *(2006.01)*

(52) Cooperative Patent Classification (CPC):
**A61K 31/409; A61K 8/00; A61K 36/02; A61P 31/04; C07D 487/22; C12P 17/10;**
A61K 2236/00; C12R 2001/89

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Université de Liège**
**4000 Liège (BE)**

(72) Inventors:
- **DAUVRIN, Thomas**
  **4000 Liège (BE)**
- **JORIS, Bernard**
  **4000 Liège (BE)**
- **FRANCK, Fabrice**
  **4000 Liège (BE)**
- **REMACLE, Claire**
  **4000 Liège (BE)**
- **AMOROSO, Ana**
  **4000 Liège (BE)**
- **GERARDS, Thomas**
  **4000 Liège (BE)**

(74) Representative: **V.O.**
**P.O. Box 87930**
**2508 DH Den Haag (NL)**

(54) **CHLOROPHYLLIDE DERIVATIVES FOR USE IN THE PREVENTION OR TREATMENT OF BACTERIAL INFECTION**

(57) The present invention relates to a compound of formula I, a stereoisomer, a tautomer and/or a dimer thereof or a salt thereof,

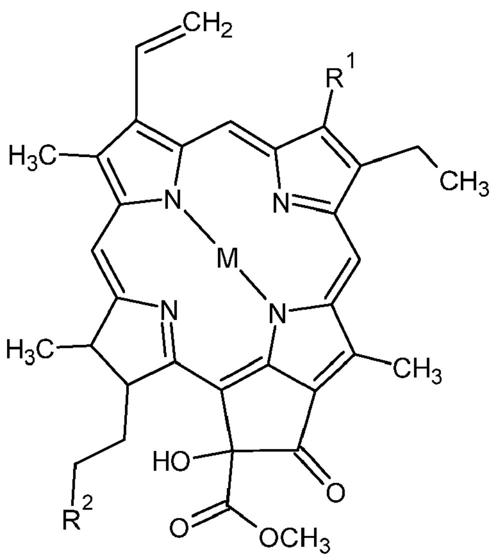

(I)

wherein M is a metal among the group of Mg, Cu and Zn; $R^1$ is a methyl group or a formyl group; $R^2$ is a carboxylic acid or a methyl ester, for use in the prevention or treatment of a bacterial infection in a subject and to the uses of such compound as antibacterial agent or particularly as preserving agents, and to processes for obtaining a microalgal extract comprising such compounds.

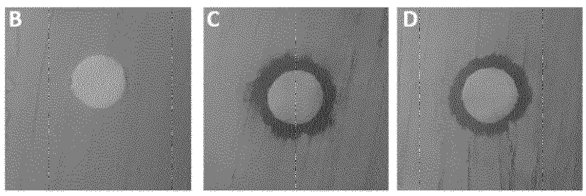


**Figure 1**

EP 4 311 550 A1

## Description

FIELD OF THE INVENTION

The invention relates to chlorophyllide derivatives for use in the prevention or treatment of a bacterial infection in a subject and to pharmaceutical compositions comprising such derivatives. The invention also relates to the use of such derivatives, to processes for preparing a microalgal extract comprising such derivatives and to the obtained microalgal extract.

BACKGROUND OF THE INVENTION

In recent years, the availability of drugs in human medicine to treat infections caused by antibiotic resistant bacteria has become increasingly problematic due to the rapid emergence of nosocomial multidrug-resistant bacteria and the lack of new antibiotics entering the market. Among these bacteria, methicillin resistant *Staphylococcus aureus* (MRSA), is responsible for around 150 000 hospital-related infections in Europe every year that leads to ~ 7 000 associated deaths (Cassini et al. Lancet Infect. Dis. (2019) 19: 56-66). Outside the hospital, S. *aureus* is a commensal organism that resides in skin and mucous membranes of humans and animals. It may be present as a nonaggressive member of the normal skin microflora or may be associated to mild illness to life-threatening sepsis that can occur if the organism enters into the body particularly in immunocompromised or immunosuppressed individual (opportunistic pathogen). Today the emergence of community-acquired MRSA is a great concern and it is important to control the overgrowth of S. *aureus,* especially the non-susceptible strains. The same holds true for other Gram-positive opportunistic pathogens such as *Enterococcus faecium* and *Enterococcus faecalis.*

Chlorophyllide *a* and chlorophyllide *b* are known as precursors of chlorophyll *a* and chlorophyll *b* respectively. Chlorophyllide may synthetically be obtained by cleavage of the phytol chain from the corresponding chlorophyll type (Scheme 1). In 1954, Blaauw-Jansen disclosed an antibacterial activity for chlorophyllides (Blaauw-Jansen, 'Chlorophyllide, the Probable Precursor of a Growth Inhibitor', Nature, 174, pp. 312-313). However, chlorophyllide disclosed by Blaauw-Jansen is only active against *S. aureus* and *B. subtilis* after being illuminated and presents no activity in the dark. In 1962, Jorgensen studied the effect of chlorophyllide extracted from microalgae (*Chlamydomonas reinhardi, Chlorella vulgaris, Scenedesmus quadricauda*) on *B. subtilis* (Jorgensen, E. G. 'Antibiotic Substances from Cells and Culture Solutions of Unicellular Algae with Special Reference to some Chlorophyll Derivatives', Physiologia Plantarum, 15, pp. 530-545). This author has again shown that chlorophyllide has no antibacterial effect when it is tested in the dark. When this compound is illuminated, inhibition zones are observed. A compound resulting from the photo-oxidation of chlorophyllide would be responsible for this antibacterial activity. Compounds very close to those found by Jorgensen were also extracted by Hansen from *Ochromonas malhamensis.* Hansen identified these compounds as products of the photo-oxidation of chlorophyllide *a*. This author has demonstrated that these derivatives are active against both Gram-positive and Gram-negative bacteria but again requiring the exposition to light (Hansen, J. A. (1973) 'Antibiotic Activity of the Chrysophyte Ochromonas malhamensis', Physiologia Plantarum, pp. 234-238.).

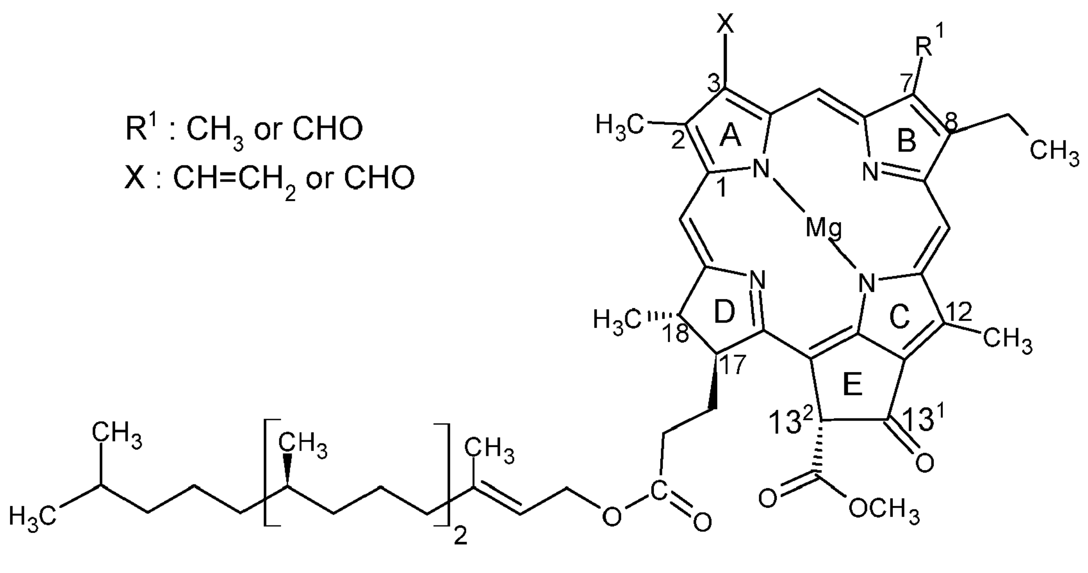

$R^1$ : $CH_3$ or CHO
X : $CH=CH_2$ or CHO

Chlorophyllide *a* :

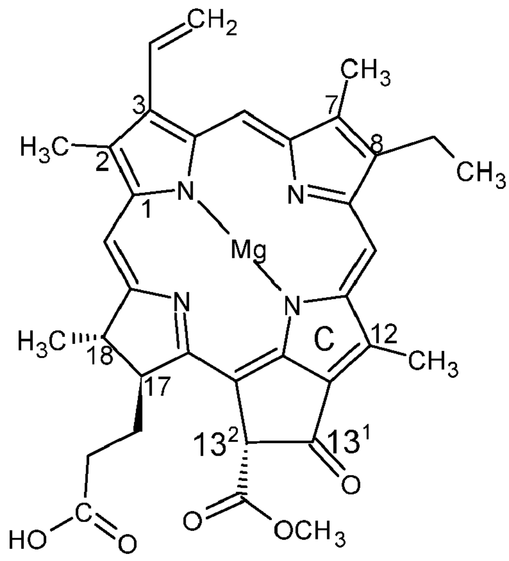

Scheme 1: Different types of chlorophyll (upper structure) and chlorophyllide *a* (lower structure)

Such chlorophyllide derivatives of the prior art, while presenting a great interest due to their natural origin (versus synthetic products) therefore lack an intrinsic antibacterial activity to be largely used for example as preserving agents in pharmaceutical or in cosmetic products. Indeed, a preserving agent should possess an antibacterial activity even in the dark so that no bacteria can develop in pharmaceutical or cosmetic products during storage in closed containers for example. There is thus still a need for compounds of natural origin presenting an antibacterial activity even in the absence of light.

It is an aim of the present invention to solve or alleviate one or more of the above-mentioned problems by providing compounds having the desired antibacterial activity. Particularly, the invention aims to provide chlorophyllide derivatives which have an antibacterial activity in the absence of light. It also aims to provide chlorophyllide derivatives which have a rapid onset of antibacterial activity. It further aims to provide chlorophyllide derivatives with a relatively low minimum inhibitory concentration.

SUMMARY OF THE INVENTION

To this aim, according to a first aspect of the present invention, there is provided a compound of formula I:

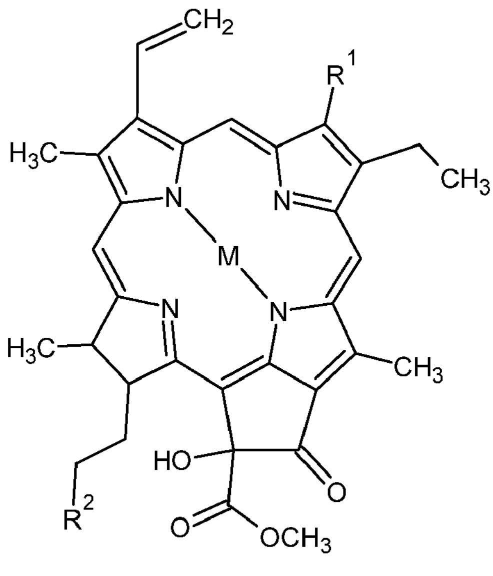

(I)

wherein M is a metal among the group of Mg, Cu and Zn; $R^1$ is a methyl group or a formyl group; $R^2$ is a carboxylic acid or a methyl ester; or

a stereoisomer, a tautomer and/or a dimer thereof; or
a salt thereof;
for use in the prevention or treatment of a bacterial infection in a subject.

The compound of formula I is a chlorophyllide derivative which bears a hydroxyl group in position $13^2$ with reference to the structure in Scheme 1 presented above in the background section. The present inventors have shown that such a group provides a compound which has a bactericidal activity. In particular, it was surprisingly shown that an inhibition of the growth of bacteria occurred in the presence of such chlorophyllide derivatives while no inhibition or only a poor inhibition of bacterial growth was observed with chlorophyllide derivatives lacking such a hydroxyl group. The difference in activity was particularly relevant when tests were performed in the dark. Hence, such derivatives surprisingly present a bactericidal activity even in the absence of light. It was also shown that with such derivatives the bacterial growth is rapidly and completely inhibited. It was further shown that the minimum inhibitory concentration is decreased with such derivatives. An antibacterial activity was shown on several Gram-positive bacteria tested. Interestingly, the compounds of the invention are thus suitable as antibacterial agents, more particularly for the prevention or treatment of diseases caused by bacterial infections. Such compounds may also be provided as part of pharmaceutical compositions for such use.

According to a further aspect of the present invention, there is provided the use of such compounds of formula I as antibacterial agents. Such use may for example be in cosmetic treatments or for example as preserving agents in cosmetic products such as to prevent bacterial growth in such products. The use may also pertain to antibacterial agents in the field of agriculture or food industry.

According to another aspect of the present invention, there is provided a process to prepare a microalgal extract comprising compounds according to formula I, to the microalgal extract obtained by such process, to the microalgal extract for use in the prevention or treatment of a bacterial infection and its use ex-vivo or in vitro as an antibacterial agent.

BRIEF DESCRIPTION OF THE DRAWINGS

Figure 1 shows inhibition diameters of S. *aureus* induced by a microalgal extract prepared according to the invention from strain *Scenedesmus sp. S4* (A is a positive control, B is a negative control, C, D and E are triplicates).
Figure 2 shows inhibition diameter of S. *aureus* induced by a microalgal extract prepared according to the invention from strain *Desmodesmus armatus S0* (A is a positive control, B is a negative control, C, D and E are triplicates).
Figure 3 shows a chromatogram from Reverse Phase HPLC on a microalgae extract prepared according to the invention, each peak representing a fraction of the extract. Inhibition diameter of S. *aureus* induced by each fraction is presented on the right.
Figure 4 shows the fluorescence emission and excitation spectra of purified S fraction.
Figure 5 shows the NMR spectrum of purified S fraction between 3.53 and 3.67 ppm. $CH_3$ protons of $13^3$ and $17^3$ methoxy are labelled with a circle and a diamond respectively.
Figure 6 shows the NMR spectrum of purified S fraction between 5.15 and 5.55 ppm. The $13^2$ hydroxy proton is labelled with a circle.
Figure 7 shows the growth curve in the dark of S. *aureus* in the presence or absence of purified S fraction, solubilized in $MeOH/H_2O$ 75% at MIC and at 4 times the MIC. The curves represent the average values of three independent experiments.
Figure 8 represents the CFU (logarithmic scale) of S. *aureus* at different growth time after addition of the purified S fraction. The negative control is represented in black, while cultures treated with 1x and 4xMIC of purified S fraction are hatched and dotted respectively. The data presented are the average values of three independent experiments.
Figure 9 represents the relative percentage of red blood cell hemolysis in liquid suspension after the addition of purified S fraction. The negative control is represented in black, red blood cells treated with 1x and 4x MIC of purified S fraction are hatched and dotted, respectively. The data presented are the average values of three independent experiments.

DETAILED DESCRIPTION OF THE INVENTION

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step

of a method is referred to as subsequent to another step, it can directly follow said other step or one or more intermediate steps may be carried out before carrying out the particular step, unless specified otherwise.

The present invention will be described with respect to particular embodiments and with reference to certain drawings but the invention is not limited thereto but only by the claims.

By metal is meant a metal according to the periodic table of elements. By metal is here particularly meant a metal suitable for the insertion in a porphyrin structure.

Porphyrin is a group of heterocyclic macrocycle organic compounds, composed of four modified pyrrole subunits interconnected at their $\alpha$ carbon atoms via methine bridges (=CH-). Metal complexes derived from porphyrin occur naturally.

By mixture is meant the presence of more than one compound or the presence of more than one variation in the structure of the compound or the presence of more than one variation in the configuration of the compound.

Chlorophyllide derivatives may present chirality, also referred to as stereoisomerism. Stereoisomers are molecules with the same molecular formula but which differ in the three-dimensional orientations of their atoms in space. An enantiomer is one of two stereoisomers that are mirror images of each other that are non-superposable. A chiral atom in a compound results in two possible structures which are non-superposable, each a mirror image of the other. The presence of multiple chiral atoms in a given compound increases the number of geometric forms possible, though there may still be some perfect-mirror-image pairs. Enantiomer are distinguished by an *R* or an S configuration according to the convention in the field.

Chlorophyllide derivatives may also present tautomerism, namely molecules with the same molecular formula but with a relocation of a hydrogen atom in the compound. In particular, tautomerism can occur for a ketone associated to an aromatic moiety. Chlorophyllide derivatives may also form dimer, namely a structure formed by the association of two sub-units, such as by the association of two porphyrin rings. The present invention includes all possible stereoisomers compounds of formula I and any subgroup thereof. When a compound is desired as a single enantiomer, such may be obtained by stereospecific synthesis, by resolution of the final product or any convenient intermediate, or by chiral chromatographic methods as each are known in the art.

The present invention pertains to a compound of formula I:

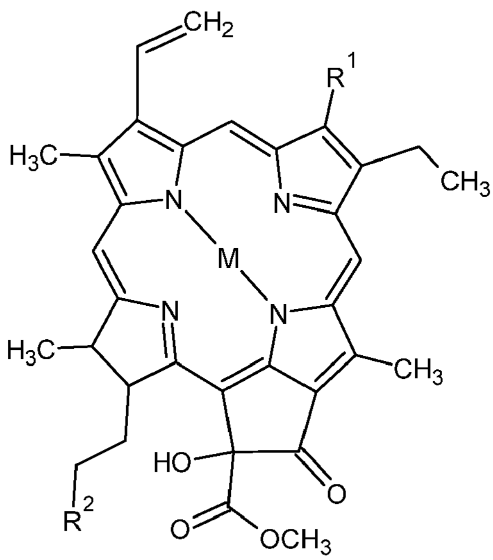

(I)

wherein M is a metal among the group of Mg, Cu and Zn; $R^1$ is a methyl group or a formyl group; $R^2$ is a carboxylic acid or a methyl ester; or

a stereoisomer, a tautomer and/or a dimer thereof; or
a salt thereof;
for use in the prevention or treatment of a bacterial infection in a subject.

The compound of formula I is a chlorophyllide derivative which bears a hydroxyl group in position $13^2$ with reference to the structure in Scheme 1 presented above in the background section. The compound of formula I may be one compound or may be a mixture of compounds of formula I. The carbon atom in position $13^2$ is a chiral carbon. The compound of formula I having an antibacterial activity may be any of the stereoisomers thereof. The carbon atom in position $13^2$ may be with an *R* or an S configuration. The compound of formula I may also be a mixture of an R and an

S configuration.

The compound of formula I is also a chlorophyllide derivative in which the group in position 7 with reference to the structure in Scheme 1 presented above in the background section, can be a methyl group or a formyl group (indicated as $R^1$ in formula I). In some cases, a mixture of a compound according to formula I bearing a methyl group in position 7 and a compound according to formula I bearing a formyl group in position 7 may be provided.

The compound of formula I is also a chlorophyllide derivative which has lost its phytol chain in position 17 with reference to the structure of chlorophyll in Scheme 1, said phytol chain being replaced either with a carboxylic acid or with a methyl ester (indicated as $R^2$ in formula I). In some cases, a mixture of a compound bearing a carboxylic acid in position 17 and a compound bearing a methyl ester in position 17 may be provided. The carbon atom in position 17 is a chiral carbon. Preferably, the configuration of original chlorophyllide is maintained, with S configuration of carbon in position 17. Hence preferred configurations are as in formula Ia and Ib:

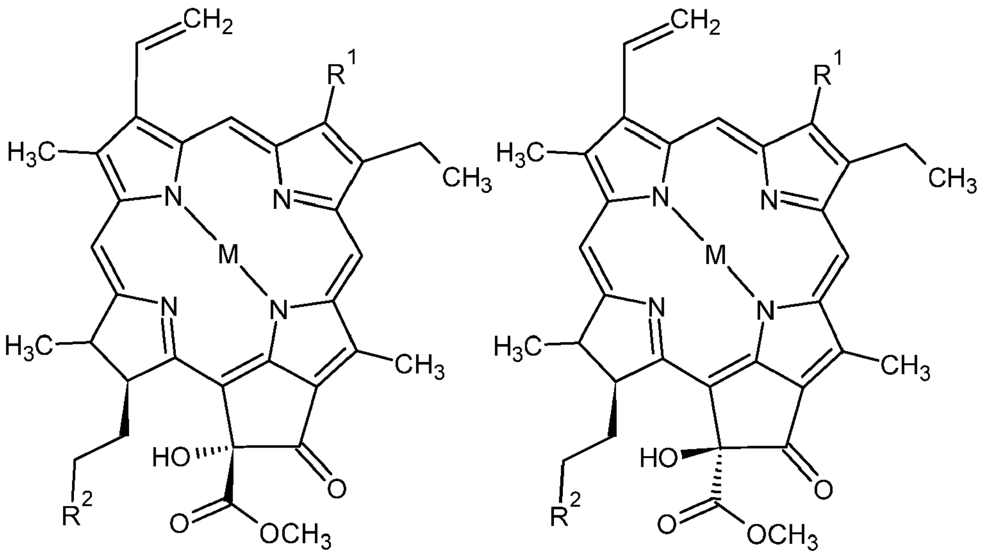

Ia Ib

The compound of formula I is also a chlorophyllide derivative which contains a metal (indicated as M in formula I) as a central position of the porphyrin structure. The metal can be Mg, Cu or Zn. Preferably the metal is Mg as in the original chlorophyllide. In some cases, a substitution of magnesium may take place with another metal, namely copper or zinc. In some cases, the substitution may be partial such that a mixture of compounds each having a different metal may be obtained, for example of mixture of compounds with two metals such as Mg and Cu, Mg and Zn, Zn and Cu or a mixture of compounds with the three metals. In some cases, the metal bears a positive charge according to its oxidation-reduction state, for example the metal is $Mg^{+2}$ or $Cu^{+2}$ or $Zn^{+2}$. A substitution by another metal may be advantageous to enhance the stability of the compound.

In some cases, the compound of formula I is in the form of a salt. Examples of salts include sodium, potassium, pyridinium and tetrabutylammonium salts.

It has been found that such compound according to formula I presents an activity for the prevention or treatment of a bacterial infection in a subject.

By bacterial infection is meant an infection caused by Gram-positive or a Gram-negative bacteria. Thus, the present invention also provides a method for the treatment of a bacterial infection in a subject in need thereof, said method comprising administering to the subject an effective amount of a compound according to formula I as described herein.

In some embodiments the subject is a mammal. In some embodiments the subject is a human. In some embodiments the subject is a human host. In particular embodiments, the infection is an infection of a bodily surface. Bodily surfaces include but are not limited to epidermis and mucous membranes. In some embodiments the surface is a bodily surface. In some embodiments the bodily surface is selected from an intact epidermal surface, a damaged epidermal surface or a mucosal surface. Damaged epidermal surface may be skin which is blistered, burnt by fire, inflamed, pustulated, sunburnt, bitten, stung or otherwise wounded. Suitable examples of mucosal surfaces include the mucosa of the mouth (including tongue), nose, eyes, throat, oesophagus, stomach vagina and rectum.

In some embodiments the bacterial infection is an infection caused by Gram-positive bacteria. Examples of Gram-positive bacteria are: *Staphylococcus aureus, Entereococcus faecium,* methicillin resistant S. *aureus, B. subtilis*

*168, Staphylococcus epidermidis, Bacillus anthracis, Corynebacterium diphtheriae, Enterococcus faecalis, Erysipelothrix rhusiopathiae, Listeria monocytogenes, Nocardia sp., Streptococcus pneumoniae, Streptococcus agalactiae* and Group A Streptococcii including *Streptococcus pyogenes* and *Clostridium sp.*

In some embodiments the bacterial infection is an infection caused by *Staphylococcus;* preferably an infection caused by *Staphylococcus aureus;* preferably an infection caused by methicillin-resistant *Staphylococcus aureus.*

Advantageously such compounds have an antibacterial activity in the absence of light, as detailed herein after in the examples. By absence of light is meant any conditions which do not allow any light ray to reach the compound nor the test plate on which the compound is present. Such conditions are for example in a dark room, a room without any window or other light source or for example in a closed container made of panels which do not allow any light ray to penetrate in the container or for example by covering directly or indirectly the test plate or a recipient containing the test plate with a light-blocking element such as an aluminium foil for example. By absence of light is also meant under illuminance value of less than 50 Lux, preferably less than 10 Lux, preferably less than 1 Lux. Advantageously, the compounds have a rapid antibacterial activity. By rapid activity is meant an activity with an onset of action in short period of time such as in a period of less than 200 minutes, preferably in less than 100 minutes, even more preferably in less than 50 minutes, even more preferably the growth of bacteria is slowed down immediately (i.e. within a period of less than 10 minutes) after addition of the compounds. Advantageously, the activity is persisting for more than two hours, preferably for more than 4 hours.

It is further advantageous that the activity is a bactericidal activity. It was indeed shown that a bacteria population could be completely killed with the compounds of the invention. By completely killed, one means that more than 99 % of the bacterial population is killed, more particularly more than 99.9 % and even more particularly more than 99.99 %.

Advantageously the compounds have a low minimum inhibitory concentration (MIC). The MIC can be lower than with other chlorophyllide derivatives.

By low MIC is meant a MIC lower than 200 μg/ml, preferably lower than 20 μg/ml, more preferably lower than 2 μg/ml. By low MIC is also meant a MIC comparable with that of antibiotics currently used. For example Vancomycin (the antibiotic used against MRSA infection) is known to have a MIC of 1.0 μg/ml.

Advantageously the compounds have no lytic effect on red blood cells.

Advantageously the compounds are soluble in aqueous and/or alcoholic solutions.

Advantageously the compounds are active on several bacteria. In particular, the compounds are active on Gram-positive bacteria. Particularly, the compounds are active on at least S. *aureus, E. faecium, methicillin resistant S. aureus and B. subtilis 168.*

A preferred compound of formula I for use in the treatment or prevention of bacterial infection is a compound wherein $R^2$ is a methyl ester. Such esterification has indeed shown an even higher antibacterial activity.

A preferred compound of formula I for use is a compound wherein $R^1$ is a methyl. Such derivative (also referred to as a chlorophyllide *a* derivative) has indeed shown an even higher antibacterial activity. Another preferred compound of formula I for use is a compound wherein the metal is magnesium (Mg). A still preferred compound for use is $13^2$-hydroxy-methylchlorophyllide *a* of formula II, wherein $R^2$ is a methyl ester, $R^1$ is a methyl and M is magnesium. Such compound surprisingly showed a high antibacterial activity compared to other compounds. Both enantiomers *S* and R presented this even higher antibacterial activity.

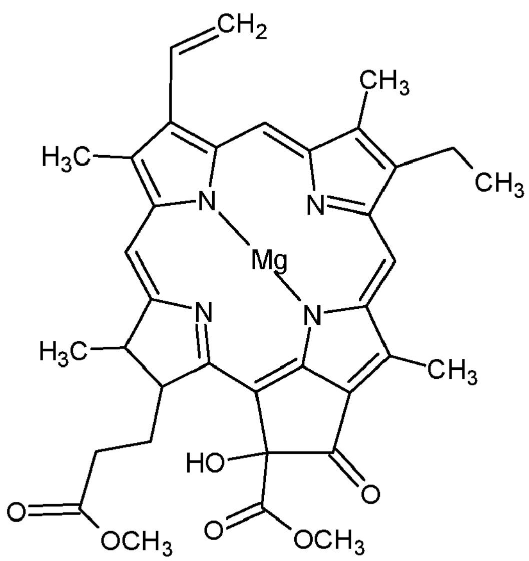

(II)

According to another aspect, there is provided a pharmaceutical composition comprising at least one compound for use as recited above. The pharmaceutical compositions may include, in addition to the compound of formula I or a mixture thereof, auxiliary substances, preservatives, solvents and/or viscosity modulating agents. By solvent, one means for example water, saline solution or any other physiological solution, ethanol, glycerol, oil such as vegetable oil or a mixture thereof.

According to yet another aspect, there is provided the use ex-vivo or in-vitro of a compound of formula I or of a mixture of compounds of formula I:

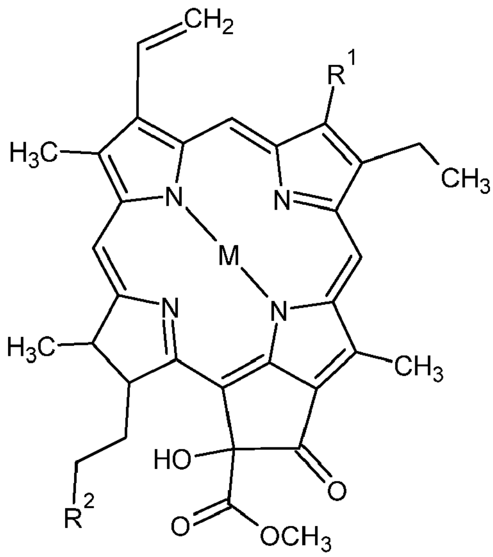

(I)

wherein M is a metal among the group of Mg, Cu and Zn; $R^1$ is a methyl group or a formyl group; $R^2$ is a carboxylic acid or a methyl ester; or

a stereoisomer, a tautomer and/or a dimer thereof; or
a salt thereof;
as an antibacterial agent.

Preferably $R^2$ in formula I is a methyl ester.

Preferably, there is provided the use of the compound which is $13^2$-hydroxy-methylchlorophyllide *a* of formula II:

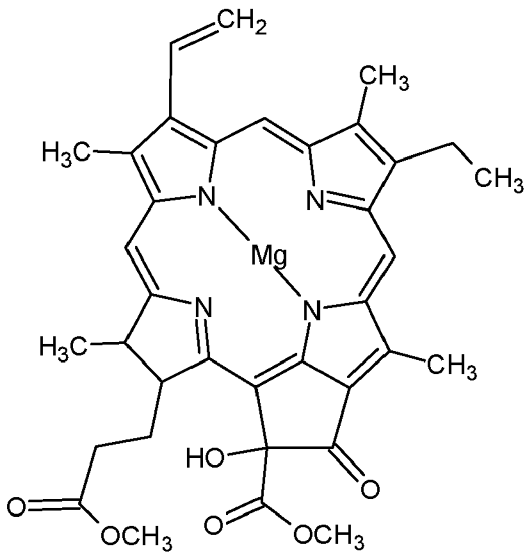

(II)

Antibacterial agents are used in different fields besides medicine, such as in cosmetics, in agriculture, in food industry. A particularly interesting use of the present compounds is the use as preserving agent (also referred to as

preservatives) in cosmetic products. Cosmetic products may be creams or gels. Particularly intended products are such products which have to be preserved from bacterial development during storage and/or use. The fact that the present compounds are active in the absence of light makes them particularly suitable for such use.

According to a further aspect, there is provided a process to prepare a microalgal extract comprising a compound of formula I:

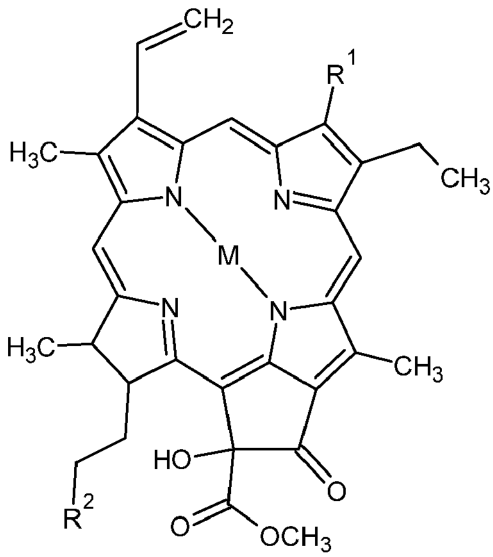

(I)

wherein M is a metal among the group of Mg, Cu and Zn; $R^1$ is a methyl group or a formyl group; $R^2$ is

a carboxylic acid or a methyl ester; or
a stereoisomer, a tautomer and/or a dimer thereof; or
a salt thereof;
said process comprising the steps of:

(i) cultivating a strain of microalgae;
(ii) drying the microalgal culture to obtain a microalgal powder;
(iii) macerating the microalgal powder in an agitated extraction solution, said extraction solution comprising methanol and water, wherein the volume ratio of methanol:water is from 3:2 to 4:1;
(iv) collecting the solution comprising the macerated microalgal extract.

Advantageously, such method steps generate the compounds of formula I without the need to add further reagents. Advantageously, such method steps generate the compounds of formula I through hydroxylation of the chlorophyll by the extraction solution. Advantageously, such method steps generate the compounds of formula I through the action of enzyme naturally occurring in microalgae strain. Advantageously, the action of enzyme is a transesterification. Advantageously, hydroxylation and transesterification reactions both take place during the method steps of present invention. Advantageously, hydroxylation and transesterification reactions both take place during the maceration step of the process according to the invention.

In an alternative embodiment, macerating the microalgae powder is carried out in an agitated extraction solution, said extraction solution comprising an alcohol and water. Said alcohol may be for example methanol, ethanol, propanol or isopropanol or a mixture thereof. The volume ratio of alcohol versus water may be from 5 vol% to 95 vol %, for example 10, 15, 20, 25, 30, 35, 40, 45, 50, 55, 60, 65, 70, 75, 80, 85, 90 vol%, preferably the volume ratio is from 60 vol% to 80 vol%, more preferably 75 vol%. Advantageously, the choice of the alcohol has an influence on the $R^2$ group of compound I obtained. When using methanol, $R^2$ may be a methyl ester. When using ethanol, $R^2$ may be ethyl ester. When using (iso)propanol, $R^2$ may be propyl ester. With an extraction solution comprising an alcohol and water, a compound of formula I with $R^2$ being carboxylic acid may also be obtained.

Suitable strains to carry out the process according to the invention include: *Scenedesmus sp., Desmodesmus sp., Ankistrodesmus sp., Chlorella sp., Euglena sp., Coelastrella sp., Haematococcus sp., Chlorella sorokinia, Desmodesmus armatus,* or any other microalgae strain containing chlorophyll esterase (generally known as chlorophyllase) in the strain. The above mentioned strains are relatively easy to cultivate, such as easy to cultivate at an industrial scale.

The strains S4 and S0 mentioned in the examples have been deposited at the Banco Espanol de Algas (BEA),

a recognised depositary institution according to the Budapest Treaty on the International Recognition of the Deposit of Microorganisms for the purposes of Patent Procedure of 28 April 1977. Following information has been mentioned:

S4, with taxonomic designation *Scenedesmus sp,* is an axenic culture, is a none genetically modified organism, is cultivated under 50 micromol.photons/$m^2$/s1, under continuous light and at 23°C, in a solid TAP medium. The storage is in day/night cycles (14h light 10 micromol photons/$m^2$/s1 and 8h dark at 16°C) and can be cryopreserved. It has no dangerous properties to health or environment to the best of our knowledge.

S0, with taxonomic designation *Desmodesmus armatus,* is an axenic culture, is a none genetically modified organism, is cultivated under 50 micromol.photons/$m^2$/s1, under continuous light and at 23°C, in a solid BOLD medium. The storage is in day/night cycles (14h light 10 micromol photons/$m^2$/s1 and 8h dark at 16°C) and can be cryopreserved. It has no dangerous properties to health or environment to the best of our knowledge.

Cultivating the strain may be by any known method of the art. Cultivation can for example be in an open outdoor system or in a bioreactor. Preferably, cultivating the strain is done in an outdoor open thin-layer cascade system. The strain could also be cultured in sterile flasks with an agitation system. The culture can be complemented with a flow of air containing $CO_2$. For example a flow of air containing about 5% of $CO_2$. The culture may be kept at room temperature. The culture is maintained under suitable light intensity. For example, the culture is maintained under a light intensity of 200 $\mu$mol photon.$m^{-2}$.$s^{-1}$. The culture is maintained in a suitable culture medium, adapted to the microalgae strain, and for a suitable duration, for example one week.

Drying the microalgae can be done by any suitable method such as atomisation, vacuum drying, flash drying, freeze drying, spray drying, lyophilisation.

Preferably, in the process according to the invention, drying the culture is performed by spray-drying. Spray-drying is a method of producing a dry powder from a liquid or slurry by rapidly drying with a hot gas. The gas can be nitrogen, argon, air or oxygen. The inlet temperature is preferably set above the boiling temperature of the extraction solution used. The inlet temperature may be between 100 and 200°C, preferably between 150°C and 180°C. The outlet temperature may be set lower than the inlet temperature, for example an outlet temperature of between 100°C and 50°C, for example 80°C. The flow rate may be between 1 ml/min and 100 ml/min, for example 25 ml/min.

It was found, as shown in the examples, that the choice of the extraction solution is important to yield the preferred compounds of formula I. Therefore, the extraction solution is preferably a mixture of methanol and water. The volume ratio of methanol:water is comprised in the range from 3:2 to 4:1. Preferably, the volume ratio is in the range from 3:2 to 3.5:1, preferably in the range from 2:1 to 3:1. Preferably, in the process according to the invention, macerating the culture is performed for at least 30 minutes, preferably for at least 1 hour, more preferably for at least 8 hours and at a temperature comprised between 0 to 60°C, preferably between 5 and 40°C, more preferably between 10 and 20°C. Such conditions may be advantageous to obtain a high yield of compounds of formula I.

Preferably, the volume of extraction solution versus the amount of algal powder is at least 5mL for 1g of algal powder, more preferably at least 10mL for 1g of algal powder, even more preferably at least 15mL for 1g of algal powder. For example, the volume of extraction solution for 1g of algal powder may be between 10mL and 1000 mL.

Preferably, in the process according to the invention, macerating the culture is performed in the absence of light. This may be advantageous to avoid a possible degradation of chlorophyll in the presence of light, such degradation being the loss of the phytol chain or possible transesterification.

Preferably, in the process according to the invention, collecting the solution comprising the macerated microalgal extract is performed through centrifugation or filtration.

Preferably, the process further comprises a step of lysing cells of the microalgae culture between step (ii) and step (iii). This lysing step may be advantageous to enhance the yield of extraction of the extract. Preferably, the process further comprises a step of enriching the microalgal extract obtained after step (iv) in one or more of the compounds of formula I. A step of enriching the microalgal extract may be the addition of a defined amount of compound of formula I to the microalgal extract obtained after step (iv). A compound of formula I may be obtained by transesterification of chlorophyllide a or b followed by hydroxylation or by any other known method.

According to a further aspect, there is provided a microalgal extract obtained by the process as recited above. Such microalgal extract may be used as an antibacterial agent directly as obtained after the process or may be purified before its use. Suitable purification may include purification by reverse HPLC or by any other chromatography method.

The invention will now be further detailed with examples showing the different aspects. It should be clear that the invention is not limited to such examples. Many alternatives are possible within the scope of protection as formulated in the claims hereafter.

EXAMPLES

Example 1: preparation of a microalgal extract by maceration

The strain S4 of *Scenedesmus* sp. was cultivated in an open door thin-layer cascade system. The culture medium used was constituted by mixing two hydroponic media, FloraBloom and FloraGro (General Hydroponics), to tap water with a ratio of 1/1000 v/v for each. The culture was then dried by spray drying to obtain a powder of microalgae. Parameters of the spray drying process were an inlet temperature of between 150°C and 180°C and an outlet temperature of 80°C. Flow rate was 25 ml/min. The microalgal powder (0.5 g of powder) was then macerated in 7.5 ml of a mixture of methanol and water 75/25 (v/v) during 18 hours at 10°C in the dark. The maceration is made on a rotating agitator. After the maceration, the mixture was centrifugated at 4250 g (g being the unit of gravitational acceleration) during 15 minutes. The supernatant (containing the extract) is transferred to a new vessel and stored at -18°C.

Example 2: preparation of a microalgal extract by maceration after lysis

A microalgal extract was prepared according to example 1, except that a lysis step was performed, so that the extract contains disrupted microalgal cells. In this case, prior to maceration, dry cells are suspended in methanol/water (75/ 25, v/v) and lysis is achieved by adding glass beads (0.75 and 1 mm) in the cell suspension. Before lysis, the vessel was kept at -18°C during 1h. The lysis (cell disruption) was obtained with the help of a Tissue Lyser II (Qiagen): two cycles of lysis during 10 minutes at 30 Hz were performed at room temperature and between the two cycles, the vessel is kept at -18°C during 15 minutes. After the lysis, the maceration took place as in example 1.

Example 3: antibacterial activity of microalgal extract

The antimicrobial activity of these extracts was tested against Gram-positive and Gram-negative bacteria, yeasts and fungi by the diffusion disk method. The bacteria tested were *Staphylococcus aureus* (ATCC 25923), *Enterococcus faecium* (ATCC 19434), *Escherichia coli* (ATCC 25922), *Pseudomonas aeruginosa* (ATCC 27853), *Klebsiella pneumoniae* (ATCC 13883), *methicillin resistant S. aureus* (MRSA, ATCC43300) and Bacillus subtilis 168. The yeasts tested were *Candida albicans* (MUCL 29800), *Saccharomyces cerevisiae* (MUCL 31497) and the fungus *Aspergillus niger* (IHEM 3415). Mueller-Hinton II culture medium was used for cultivating *S.aureus, E. coli, P. aeruginosa and K. pneumoniae* ; Brain Heart infusion Broth for *E. faecium* ; Yeast Peptone Dextrose Broth for C. *albicans* and *A. niger* and S. *cerevisiae.* Agar plates with the appropriate medium were inoculated from the glycerol stock culture and Petri dishes were incubated overnight at 37°C for bacteria and at 28°C during 48 hours for the other strains. Colonies or spores (forA. *niger*) were then resuspended in a sterile solution of NaCl 0.85% to obtain a OD600 between 0.08 and 0.1 at 600 nm (which corresponds to tube of 0.5 on McFarland scale). Sterile cotton buds were soaked with the suspension and the micro-organisms were spread onto the Petri dish. Five sterile disks of chromatography paper (Whatman 3MM) with a diameter of 6,0 mm were placed on the agar plate. Two disks were respectively used for positive (1 mg/ml kanamycin for S. *aureus, B. subtilis, E. coli* and *K. pneumoniae;* 25 mg/ml kanamycin for *E. faecium;* 0,01 mg/ml ciprofloxacin for P. aeruginosa; 20 and 0,01 mg/ml cycloheximide for *A. niger* and S. *cerevisiae,* respectively; 0,1 mg/ml nystatin for C. *albicans*) and negative ($MeOH/H_2O$ 75/25%) controls. The remaining disks were used to test the compound of interest in triplicate. On each disk, 10 μl were loaded on the paper disk in two steps (2 x 5 μl) letting the paper dry between the two loadings. The Petri dishes were then incubated as described above. The antimicrobial activity of compounds of interest was visualized by its growth inhibition halo. Both macerates (without or with cell lysis) showed an antibacterial activity against the four selected Gram-positive bacteria namely S. *aureus* (ATCC 25923), *E. faecium* (ATCC 19434), MRSA (ATCC43300), and *B. subtilis 168.* Inhibition diameters of strains S4 and S0 are presented in Figure 1 and 2 respectively.

Table 1: Inhibition diameter of the crude methanol 75% extract of spray-dried powder of *Scenedemus* sp. S4 and freeze-dried powders of *D. armatus* S0

| **Strain** | **Inhibition diameter (cm)** | | | | |
|---|---|---|---|---|---|
| | Positive control | Negative control | Repetition 1 | Repetition 2 | Repetition 3 |
| *Scenedesmus* sp. S4 | 2.10 | 0.60 | 1.12 | 1.08 | 1.16 |
| *D. armatus* S0 | 1.62 | 0.60 | 0.81 | 0.88 | 0.85 |

Example 4: purification and identification of active compounds in the microalgal extract

To identify the active compounds, the crude extract was separated by reverse HPLC (Waters e2695) with a C18 column (Nucleodur C18 Gravity EC, 3 μm particle size, length x internal diameter: 25 cm x 4.6 mm). The column was eluted by successive linear gradients of $NH_4HCO_3$ 25mM pH7 (buffer A) and acetonitrile (buffer B - indicated as ACN) and the flow rate set at 0.5 ml/min. The following table presents the mobile phase gradient profile used in elution.

Table 2: mobile phase gradient during HPLC elution

| Time (min) | % ACN |
|---|---|
| 0-5 | 5 % |
| 5-8 | 50% |
| 8-13 | 50% |
| 13 -49 | 100 % |
| 49-54 | 100 % |
| 54-55 | 5 % |
| 55-60 | 5 % |

The injection volume was fixed at 100 μL, the sample temperature in the autosampler was 10°C and the column was thermostatized at 37°C. The effluent was monitored by a photodiode array detector (Waters 2998 PDA detector, detection range: 200-800 nm) and collected using an automatic fraction collector (Waters Fraction Collector III, volume fraction: 500 μl). The collected fractions were then dried by using a vacuum centrifuge (Speedvac vacuum concentrator Savant SPD-111V, Thermo Scientific) and redissolved in 500 μL of $MeOH/H_2O$ 75% (75/25 v/v) and assayed for their capacity to inhibit the growth of S. *aureus* (disk assay as described above). Several fractions were selected for their antibacterial activity and preparative purifications were then carried out to obtain higher quantities of these compounds to allow their identification and subsequently their characterization.

Preparative chromatography was carried out by using an AKTA explorer system (GE Healthcare) and a preparative reverse phase column (Nucleodur C18 Gravity, 5 μm particule size, length x internal diameter: 25 cm x 1 cm) The chromatogram was recorded at 3 different wavelengths: 230 nm, 430 nm and 650 nm. The volume injected was 2 ml and the flow rate fixed at 2 ml/min. The same buffers as those used during analytical separation were selected and the gradient adapted to the column volume. Fraction size was adapted in such a way that one peak corresponded to one collected fraction. These fractions were then dried with a vacuum centrifuge and redissolved in 500 μL of $MeOH/H_2O$ 75%. Figure 3 presents the chromatogram obtained at 430 nm. Each peak is labelled with a letter and each collected compound is labelled with the corresponding letter. Figure 3 also presents the disk diffusion assays on the different compounds, for their activity of inhibition of S. *aureus* growth. The most active fraction is the S fraction. Other active fractions are noted, in particular fractions labelled C, E, H, J, K, L, and R.

Fractions were characterized by UV-Vis/Fluorescence spectroscopy, mass spectrometry and NMR. The fluorescence spectrum for compound S is presented in Figure 4 while the NMR spectra of compound S are presented in Figures 5 and 6. In particular, Figure 5 shows the NMR spectrum of purified S fraction between 3.53 and 3.67 ppm. $CH_3$ protons of $13^3$ and $17^3$ methoxy are labelled with a circle and a diamond respectively. Figure 6 shows the NMR spectrum of purified S fraction between 5.15 and 5.55 ppm. The $13^2$ hydroxy proton is labelled with a circle.

The exact masses of the molecule present in C, E, H, J, K, L, R and S fractions as well as their fragments generated by collision-induced dissociation, were determined by electrospray ionization mass spectrometry coupled to ultra-performance liquid chromatography (UPLC-ESI-MS/MS) on a Q-exactive Plus apparatus (Thermofisher). The column and the solvent used for this analysis were the same as those used for the analytical HPLC experiment, except that the column was not in an oven and the gradient adapted to ESI-MS/MS analysis. The structure of these compounds was determined by combining the molecular formula deduced by mass spectrometry, UV-Visible/fluorescence spectroscopy and with the fragmentation patterns obtained during MS/MS experiments.

The compounds present in C, E, H, J, K, L, R and S fractions are all chlorophyllide derivatives bearing a hydroxyl at position $13^2$. This latter result is consistent with the fact that a loss of 18,01 Da, corresponding to $H_2O$, was observed in the MS/MS patterns for all of purified molecules. The names and the exact masses of the active compounds present in these fractions of microalgal extract prepared according to example 1 are summarized in the following table.

Table 3

| Peak | Exact Mass (g/mol) | Molecular formula | Name of active compound |
|---|---|---|---|
| c | 644.21131 | $C_{35}H_{32}O_7N_4Mg$ | $13^2$-hydroxy-chlorophyllide *b* |
| E | 644.21172 | $C_{35}H_{32}O_7N_4Mg$ | $13^2$-hydroxy-chlorophyllide *b* |
| H | 630.23236 | $C_{35}H_{34}O_6N_4Mg$ | $13^2$-hydroxy-chlorophyllide *a* |
| J | 630.23259 | $C_{35}H_{34}O_6N_4Mg$ | $13^2$-hydroxy-chlorophyllide *a* |
| K | 658.22662 | $C_{36}H_{34}O_7N_4Mg$ | $13^2$-hydroxy-methylchlorophyllide *b* |
| L | 658.22692 | $C_{36}H_{34}O_7N_4Mg$ | $13^2$-hydroxy-methylchlorophyllide *b* |
| R | 644.24740 | $C_{36}H_{36}O_6N_4Mg$ | $13^2$-hydroxy-methylchlorophyllide *a* |
| S | 644.24797 | $C_{36}H_{36}O_6N_4Mg$ | $13^2$-hydroxy-methylchlorophyllide *a* |

Example 5: antibacterial activity of $13^2$-hydroxy-chlorophyllide derivatives

The specific activity of $13^2$-hydroxy-chlorophyllide derivatives was determined by disk assay and estimated as follows. The specific activity is the ratio between the inhibition diameter and the logarithm concentration of the chlorophyllide derivatives. This has been determined by analytical HPLC and by calculating the peak area at 430 nm or 460 nm for chlorophyllide *a* and *b* derivatives, respectively. This allowed the comparison of antibacterial activity of the active molecules regardless of their different concentration of their stock solutions. After incubation in the dark, the most active compounds were the two $13^2$-hydroxy-methyl derivatives, with the chlorophyllide *a* derivatives being the most active. When the Petri dishes were incubated in presence of light, the antibacterial activity was increased, without modifying the order of activity of the compounds. The inhibition diameters are also included in brackets.

Table 4

| Name of compound | specific activity in the dark | specific activity in presence of light |
|---|---|---|
| $13^2$-hydroxy-chlorophyllide *a* | + (0.69 cm) | +++ (0.86 cm) |
| $13^2$-hydroxy-chlorophyllide *b* | + (0.72 cm) | + (0.70 cm) |
| $13^2$-hydroxy-methylchlorophyllide *a* | ++ (0.80 cm) | ++++ (1.12 cm) |
| $13^2$-hydroxy-methylchlorophyllide *b* | + (0.73 cm) | +++ (0.87 cm) |
| methyl chlorophyllide *a* (comparative example) | - (0.60 cm) | |

Example 6: Influence of extraction solution during maceration step

A microalgal extract was prepared as described in example 1 except that different extraction solutions were tested. In particular, different methanol/water ratio were tested, as well as different alcohols. The presence of the compound $13^2$-hydroxy-methylchlorophyllide *a* was then assessed in each sample. Results are presented in the following table and highlight the importance of selecting a suitable extraction solution to obtain the desired compound. It was speculated that the presence of water is important for the transesterification to take place. The preferred ratio is a ratio of 75/25 of volume of methanol versus volume of water. The extraction with ethanol or isopropanol did not yield $13^2$-hydroxymethylchlorophyllide a compound.

Table 5

| Methanol/$H_2O$ % (v/v) | Presence of $13^2$-hydroxy-methylchlorophyllide *a* |
|---|---|
| 25/75 | - |
| 50/50 | - |
| 60/40 | + |
| 75/25 | +++++ |
| 90/10 | - |

(continued)

| Methanol/$H_2O$ % (v/v) | Presence of $13^2$-hydroxy-methylchlorophyllide *a* |
|---|---|
| 100/0 | - |

Example 7: characterization of antibacterial activity of $13^2$-hydroxy-methylchlorophyllide *a*

The minimum inhibitory concentration (MIC) of $13^2$-hydroxy-methylchlorophyllide *a* for *S. aureus* was determined using the microdilution method. In a 48-well plate, 500 μl of Mueller-Hinton broth were placed in each well. Then, 500 μl of a dilute solution of $13^2$-hydroxy-methylchlorophyllide *a* (stock solution in MeOH/$H_2O$ 75% diluted 5x by Mueller-Hinton broth) were added to the first well and serial 2-fold dilutions were made by transferring 500 μL from one well to the next. For the last well, 500 μl were discarded. A culture of *S. aureus* (ATCC25923) growing in exponential phase was diluted with sterile NaCl 0.85% (w/v) to obtain a bacterial suspension corresponding to 0.5 tube McFarland unit and then dispensed in 500 μl aliquots in the 48 well plate. After covering the 48-plate with its lid, it was incubated at 37°C under agitation and in the dark for 24 hours. The MIC was defined as the last dilution for which no visible bacterial growth was observed. The concentration of the active compound was determined on the basis of a calibration line obtained by injecting increasing volumes of a solution of pure chlorophyllide *a* standard on a reverse-phase HPLC column. The concentration of $13^2$-hydroxy-methylchlorophyllide *a* stock solution was 58.03 μg/ml and the MIC value of $13^2$-hydroxy-methylchlorophyllide *a* was 1.45 μg/ml (1:40 dilution factor) for S. *aureus.*

To further characterize the antibacterial action of $13^2$-hydroxy-methylchlorophyllide *a,* a S. *aureus* culture in its early exponential phase of growth was submitted to the action of the chlorophyllide derivative. 100 ml of Mueller-Hinton broth were inoculated with an overnight culture of S. *aureus* to obtain a bacterial density corresponding to an optical density at 600 nm ($OD^{600}$) = 0.05. This culture was then incubated at 37°C under orbital shaking (240 rpm) by using an Aquatron (Infors) shaker. Once the cells enter early exponential growth ($OD^{600}$= 0.2), the culture was subdivided into three 20 ml cultures. For two cultures, $13^2$-hydroxy-methylchlorophyllide *a* solubilized in MeOH/$H_2O$ 75% was added to reach a final concentration equal to 1x and 4x the MIC, respectively. For the third culture (control culture), a corresponding volume of MeOH/$H_2O$ without any chlorophyllide compound was added. In Figure 7 the optical density as a function of time is presented. The addition of the compounds occurred after 75 minutes. As presented on this figure, it was observed that directly after the addition of $13^2$-hydroxy-methylchlorophyllide *a, S. aureus* growth was stopped, and this for the two concentrations tested. $OD^{600}$ remained unchanged during the whole experiment. This observation supports the hypothesis that no bacterial cell lysis takes place and that the antibacterial activity of $13^2$-derivative is either bacteriostatic or non-lytic bactericidal.

To distinguish between these two antibacterial activities, the number of viable S. *aureus* were determined by counting the colonies forming units (CFU) per ml of culture before the addition of the chlorophyllide derivative (t=0 h) and after 2 and 4 hours. Figure 8 shows the CFU in logarithmic scale of S. *aureus* at different growth time after addition of the purified S fraction. The negative control is represented in black, while cultures treated with 1x and 4xMIC of purified S fraction are hatched and dotted respectively. The data presented are the average values of three independent experiments.

For culture without addition of chlorophyllide derivative (negative control, only addition of MeOH/$H_2O$ 75%) S. *aureus* maintained its growth and CFU/ml increased. On the contrary, once $13^2$-hydroxy-methylchlorophyllide *a* was added (1 or 4 x MIC value in MeOH/$H_2O$ 75%, final concentration), the number of CFU/ml decreased of at least four logarithmic steps (4 $log_{10}$). This means that the chlorophyllide derivative killed, in the dark, more than 99.99% of the bacterial population. The same result was observed after 2 and 4 hours even if for this latter culture a slight return of bacterial growth was observed. From these results, it may be concluded that $13^2$-hydroxy-methylchlorophyllide *a* had a rapid non-lytic bactericidal activity.

Example 8: Lytic activity of $13^2$-hydroxy-methylchlorophyllide *a* on red blood cell

Activity of $13^2$-hydroxy-methylchlorophyllide *a* on red blood cell was determined using a sheep erythocyte lysis assay. The lytic action was assessed on defibrinated sheep blood (bioTRADING). The erythrocytes were harvested by centrifugation (1500 rpm, 5 min) and washed 10 times with phosphate buffer saline (PBS). The red blood cells were then diluted 25 times with PBS and washed two more times to obtain a colourless supernatant after a centrifugation at 3000 g for 5 minutes. Samples of 1 ml of red blood cells (25 times diluted) are transferred in Eppendorf tubes and the $13^2$-hydroxy-methylchlorophyllide *a,* dissolved in MeOH/$H_2O$ 75% (v/v), was added to obtain a final concentration equal to 1x or 4x MIC. The tubes are then incubated at 37°C at 300 rpm by using a thermomixer HC (Starlab) and 100 μl samples were withdrawn at time 0, 5, 10, 15, 30, 60 and 120 min. PBS and MeOH/$H_2O$ 75% (volume added equal to sample volume) were used as negative controls whereas 0.1% sodium dodecyl sulfate (SDS) was used as positive control.

The erythrocyte lysis was highlighted by the presence of haemoglobin in the supernatant and quantified by measuring the absorbance of the supernatant at 540 nm by means of a microplate reader Tecan infinite200 pro. The percentage lysis was calculated as follows:

$$\%\,\text{lysis} = \frac{A_{Chl}^{540} - A_{PBS}^{540}}{A_{SDS\ 0.1\%}^{540}} \times 100$$

wherein $A_{Chl}$ is the absorbance of the tested compound, $A_{PBS}$ is the measured absorbance of the PBS solution and $A_{SDS}$ is the absorbance of the SDS solution (positive control), each at the wavelength of 540 nm. Figure 9 shows the percentage (calculated with the above formula) of red blood cell hemolysis in liquid suspension after the addition of purified S fraction. The negative control is represented in black, red blood cells treated with 1x and 4x MIC of purified S fraction are hatched and dotted, respectively. The data presented are the average values of three independent experiments.

The MeOH/$H_2O$ 75% had no lytic effect on the erythrocytes. This was also the case for $13^2$-hydroxy-methyl-chlorophyllide *a* for the two concentrations tested for which a percentage lower than 10 % was observed after 120 min and at 4 x MIC. The histograms are presented in Figure 9. It is commonly accepted that a compound is not haemolytic when the percentage of hemolysis is below 10% (Amin & Dannenfelser (2006) 'In Vitro Hemolysis: Guidance for the Pharmaceutical Scientist', Journal of Pharmaceutical Sciences, 95(6), pp. 1173-1176).

## Claims

1. Compound of formula I:

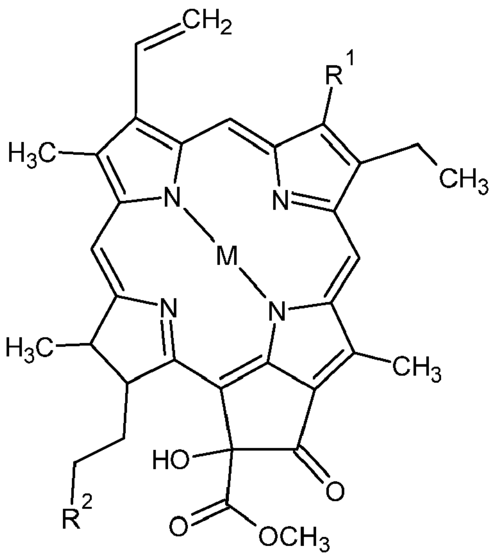

(I)

wherein M is a metal among the group of Mg, Cu and Zn; $R^1$ is a methyl group or a formyl group; $R^2$ is

a carboxylic acid or a methyl ester; or
a stereoisomer, a tautomer and/or a dimer thereof; or
a salt thereof;
for use in the prevention or treatment of a bacterial infection in a subject.

2. Compound for use according to claim 1, wherein $R^2$ is a methyl ester.

3. Compound for use according to any of previous claims which is $13^2$-hydroxy-methylchlorophyllide *a* of formula II:

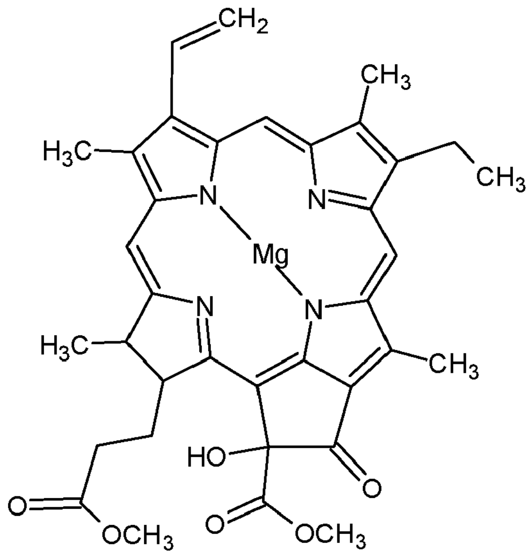

(II)

4. A pharmaceutical composition comprising at least one compound for use according to any of previous claims and a pharmaceutically acceptable diluent.

5. Use ex-vivo or in-vitro of a compound of formula I or a mixture of compounds of formula I:

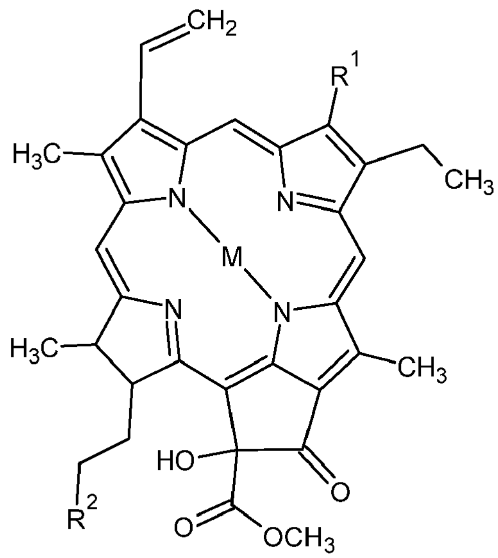

(I)

wherein M is a metal among the group of Mg, Cu and Zn; $R^1$ is a methyl group or a formyl group; $R^2$ is

a carboxylic acid or a methyl ester; or
a stereoisomer, a tautomer and/or a dimer thereof; or
a salt thereof;
as an antibacterial agent.

6. Use according to claim 5 wherein $R^2$ is a methyl ester.

7. Use according to any of claims 5 to 6 wherein the compound is $13^2$-hydroxy-methylchlorophyllide *a* of formula II:

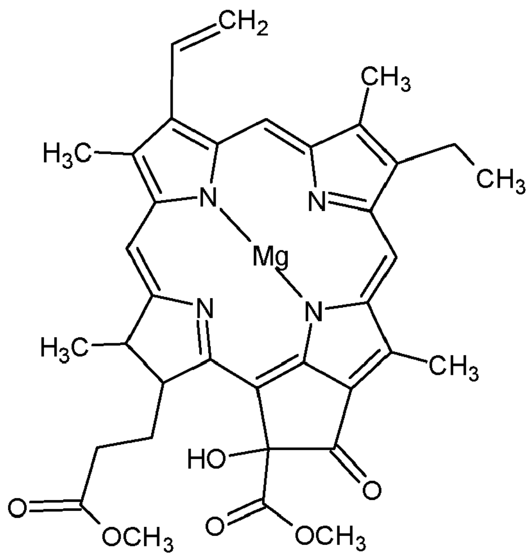

(II)

8. Use according to any of claims 5 to 7 as preserving agent in a cosmetic product.

9. Process to prepare a microalgal extract comprising a compound of formula I:

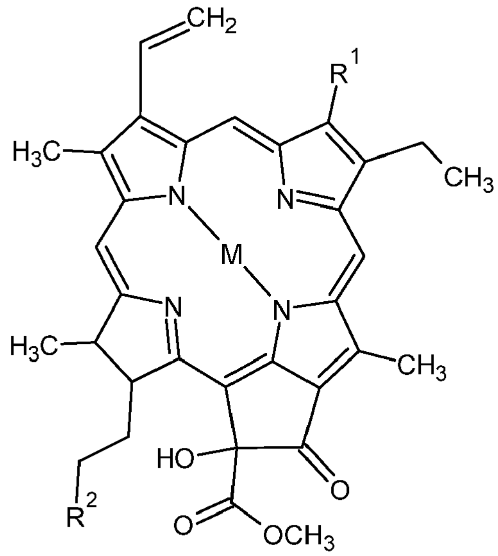

(I)

wherein M is a metal among the group of Mg, Cu and Zn; $R^1$ is a methyl group or a formyl group; $R^2$ is

a carboxylic acid or a methyl ester; or
a stereoisomer, a tautomer and/or a dimer thereof; or
a salt thereof;
said process comprising the steps of:

(i) cultivating a strain of microalgae;
(ii) drying the microalgae culture to obtain a microalgal powder;
(iii) macerating the microalgal powder in an agitated extraction solution, said extraction solution comprising methanol and water, wherein the volume ratio of methanol:water is from 3:2 to 4:1;
(iv) collecting the solution comprising the macerated microalgal extract.

10. Process according to claim 9 wherein drying the culture is performed by spray-drying.

11. Process according to any of claim 9 to 10 further comprising a step of lysing cells of the microalgae culture between

step (ii) and step (iii).

**12.** Process according to any of claim 9 to 11 further comprising a step of enriching the microalgal extract obtained after step (iv) in one or more of the compounds of formula (I).

**13.** Microalgal extract obtainable by the process according to any of claim 9 to 12.

**14.** Microalgal extract as defined in claim 13 for use in the prevention or treatment of a bacterial infection in a subject.

**15.** Use ex-vivo or in vitro of a microalgal extract as defined in claim 13 as an antibacterial agent.

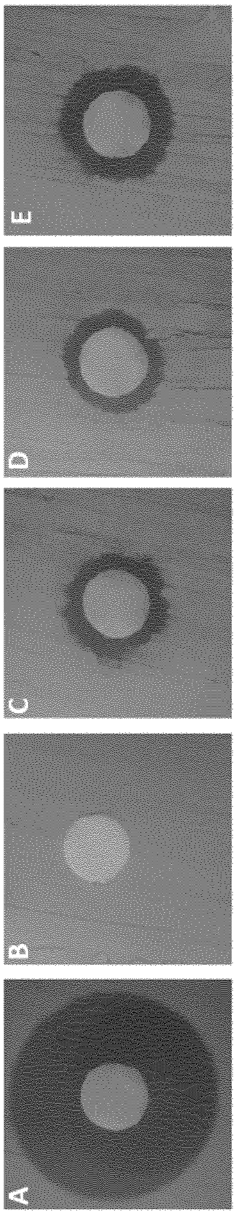


Figure 1

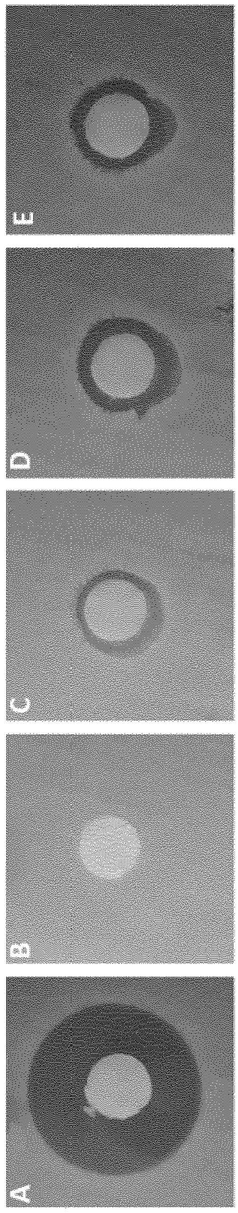


Figure 2

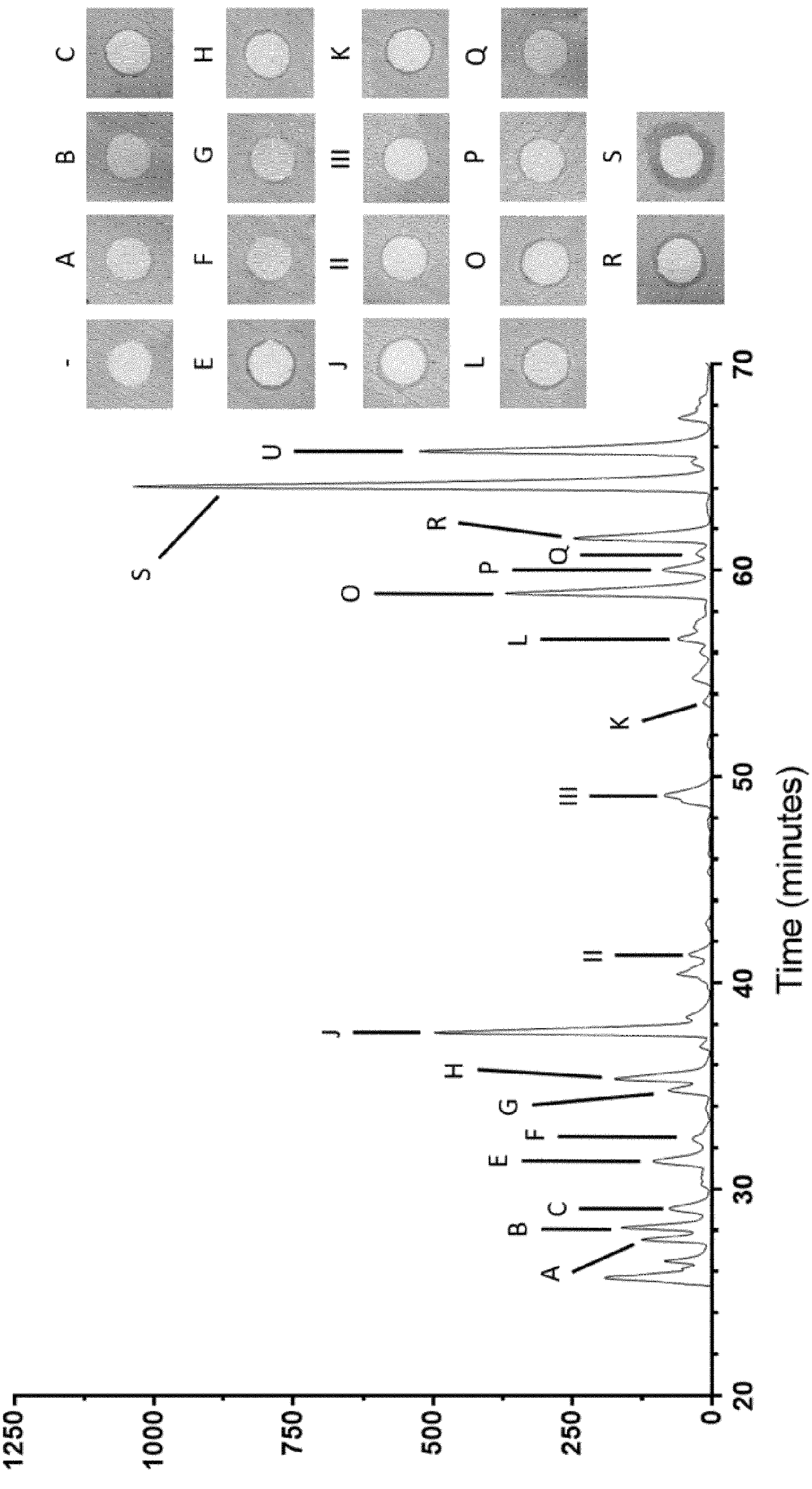
Absorbance 430nm (mAU)
Time (minutes)
A
B
C
E
F
G
H
J
II
III
K
L
O
P
Q
R
S
U
-

Figure 3

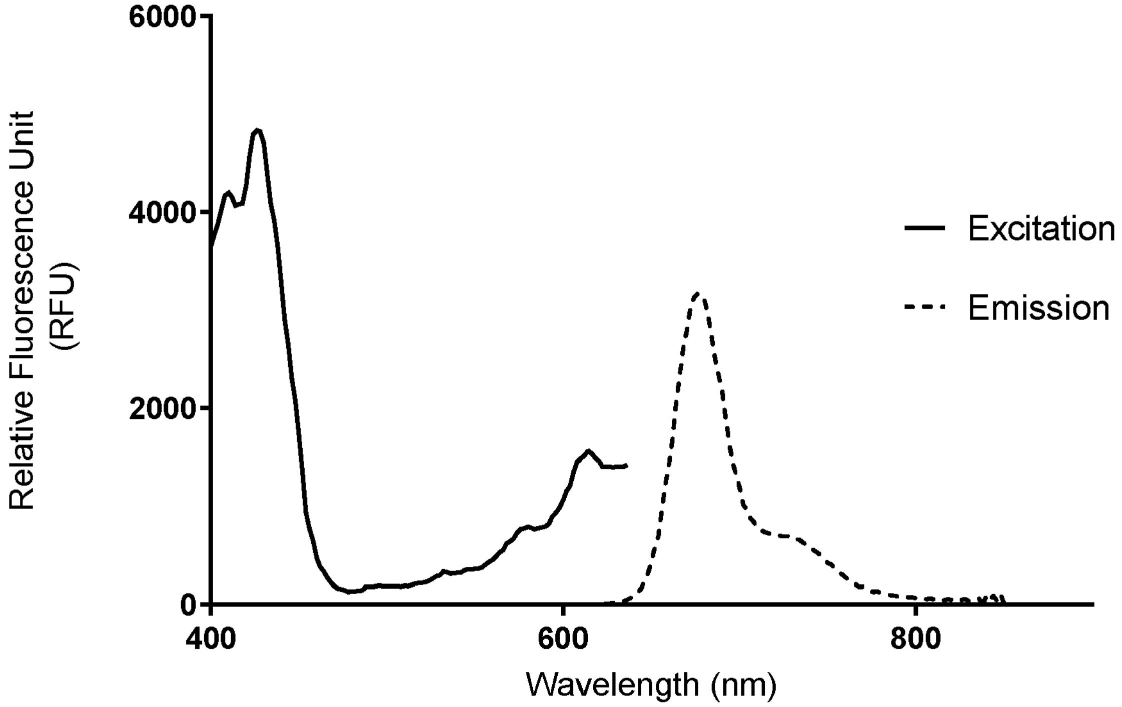


Figure 4

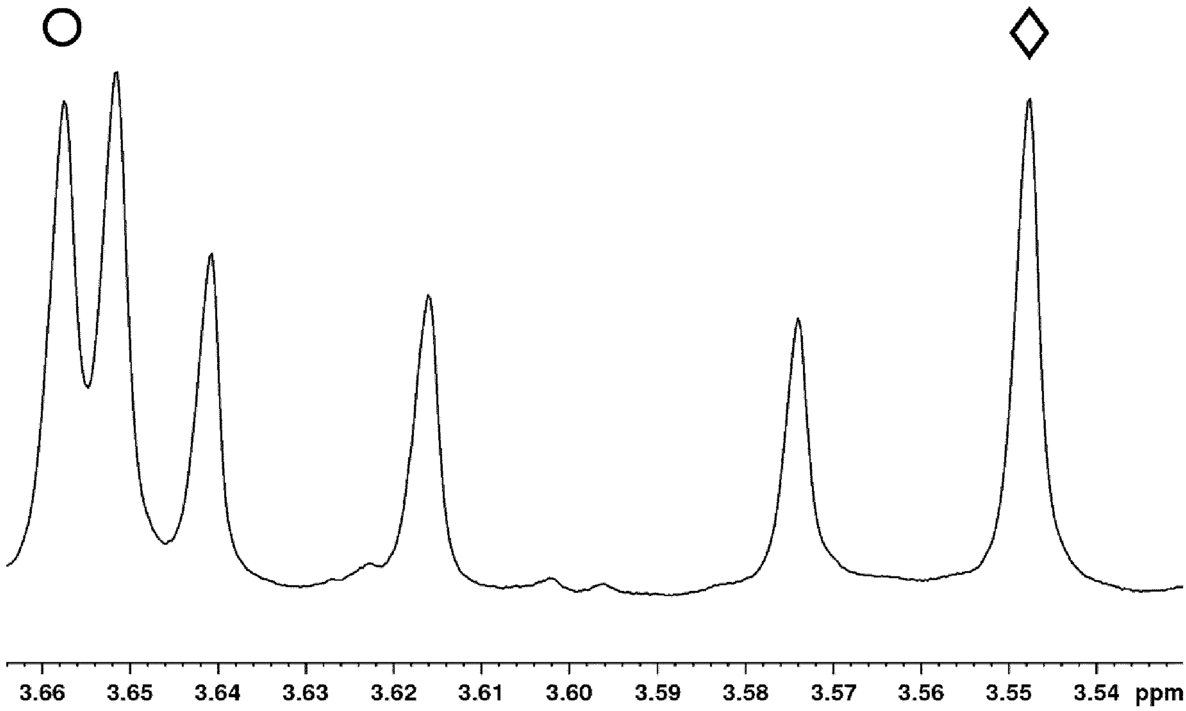


Figure 5

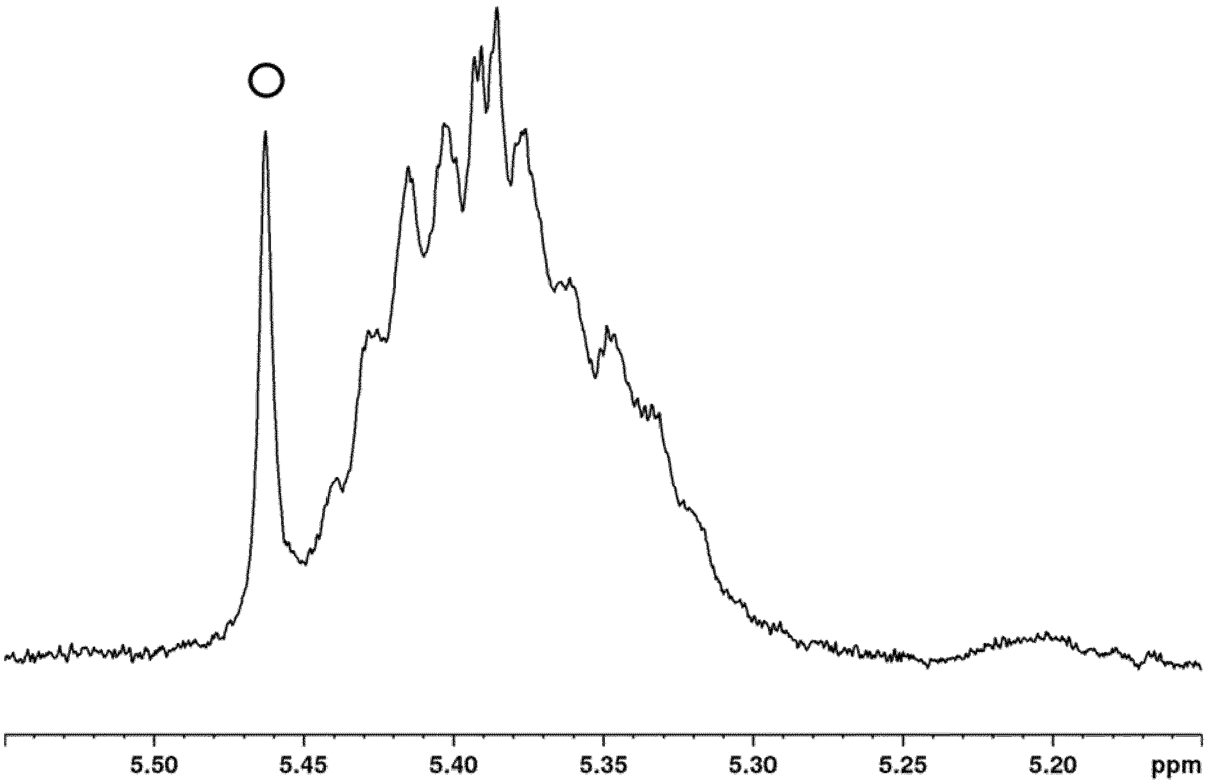


Figure 6

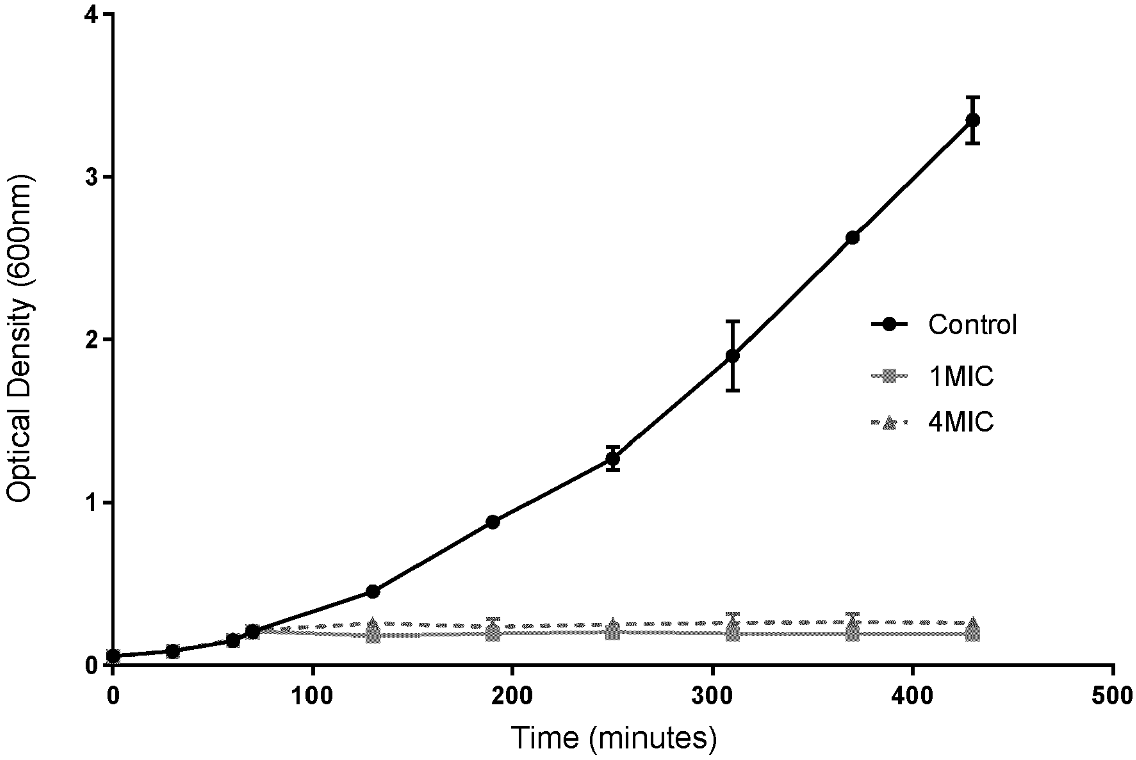


Figure 7

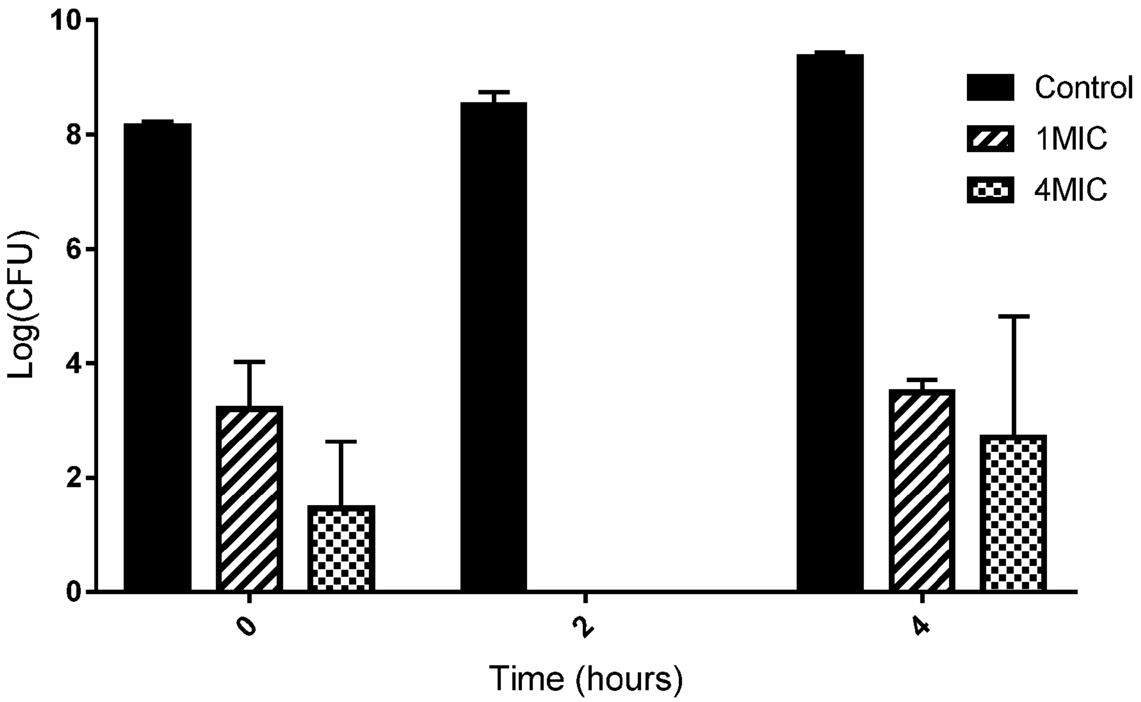


Figure 8

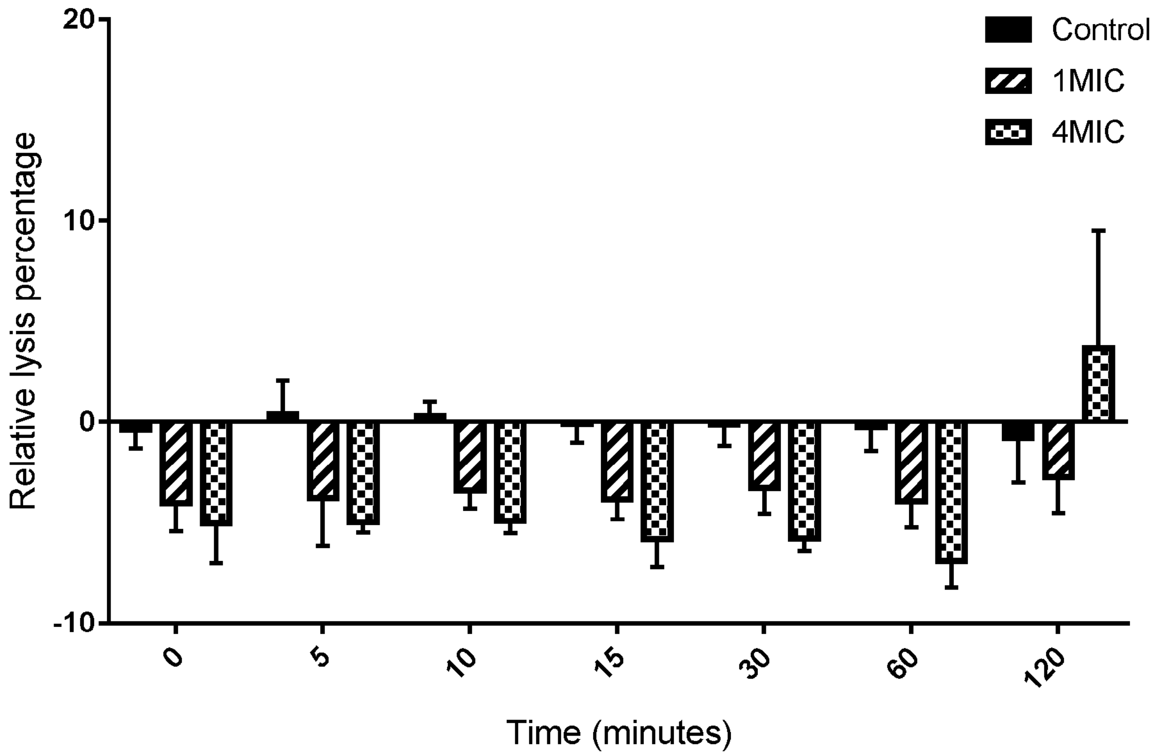


Figure 9

Europäisches Patentamt
European Patent Office
Office européen des brevets

# EUROPEAN SEARCH REPORT

Application Number
EP 22 18 7606

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GOMES ROOSEVELT ALBUQUERQUE ET AL: "Phytoconstituents from Sidastrum micranthum (A. St.-Hil.) Fryxell (Malvaceae) and antimicrobial activity of pheophytin a", BRAZILIAN JOURNAL OF PHARMACEUTICAL SCIENCES, vol. 51, no. 4, 1 December 2015 (2015-12-01), pages 861-867, XP093010021, DOI: 10.1590/S1984-82502015000400012 * second column, first section, table 1; page 865 * * page 864; figure 4 * * conclusion; page 866 * | 1-15 | INV. A61K31/409 A61K8/00 A61K36/00 A61P31/04 C07D487/00 C12N1/00 |
| X | ALSENANI FAISAL ET AL: "Evaluation of microalgae and cyanobacteria as potential sources of antimicrobial compounds", SAUDI PHARMACEUTICAL JOURNAL, ELSEVIER, AMSTERDAM, NL, vol. 28, no. 12, 26 November 2020 (2020-11-26), pages 1834-1841, XP086426892, ISSN: 1319-0164, DOI: 10.1016/J.JSPS.2020.11.010 [retrieved on 2020-11-26] * section 2.3, extraction methods; page 1836 * | 9-12 | TECHNICAL FIELDS SEARCHED (IPC) A61K C12R A61Q C07D A61P C12N |
| A | KR 2020 0078109 A (UNIV NAT INCHEON RES & BUSINESS FOUND [KR]) 1 July 2020 (2020-07-01) * paragraph [0001] * * abstract * | 1-15 | |

-/--

The present search report has been drawn up for all claims

| Place of search | Date of completion of the search | Examiner |
|---|---|---|
| Munich | 22 December 2022 | Werner, Doris |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 18 7606

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BRUCE D. L. ET AL: "The Identification of Two Antibacterial Products of the Marine Planktonic Alga Isochrysis galbana", JOURNAL OF GENERAL MICROBIOLOGY, vol. 48, no. 2, 1 August 1967 (1967-08-01), pages 293-298, XP093008541, GB ISSN: 0022-1287, DOI: 10.1099/00221287-48-2-293 * section methods; page 293 * * page 295; figure 1 * * abstract * * page 295; table 1 * ----- | 1-8, 13-15 | |
| A | Scheer H. ET AL: "STRUCTURE OF METHYLPHEOPHORBIDE-RCI", Photochemistry and photobiology, 1 May 1986 (1986-05-01), pages 559-571, XP093009456, Oxford, UK DOI: 10.1111/j.1751-1097.1986.tb09535.x Retrieved from the Internet: URL:https://onlinelibrary.wiley.com/doi/10.1111/j.1751-1097.1986.tb09535.x [retrieved on 2022-12-19] * second column, section, 13-Hydroxy-20-chloro-pheophytin a; page 561 * ----- | 1-15 | TECHNICAL FIELDS SEARCHED (IPC) |

-/--

The present search report has been drawn up for all claims

| Place of search | Date of completion of the search | Examiner |
|---|---|---|
| Munich | 22 December 2022 | Werner, Doris |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 4

Europäisches Patentamt
European Patent Office
Office européen des brevets

# EUROPEAN SEARCH REPORT

Application Number
EP 22 18 7606

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HELENA M AMARO ET AL: "Antimicrobial activities of microalgae: an invited review", SCIENCE AGAINST MICROBIAL PATHOGENS: COMMUNICATING CURRENT RESEARCH AND TECHNOLOGICAL ADVANCES, 1 January 2011 (2011-01-01), pages 1272-1280, XP055410064, * page 1273 - page 1275 * * page 1274; table 1 * ----- | 1-15 | |
| A | US 2006/128683 A1 (WOO NAM-TAE [KR] ET AL) 15 June 2006 (2006-06-15) * claims * ----- | 1-15 | |
| A | WO 2020/178713 A1 (CIIMAR CENTRO INTERDISCIPLINAR DE INVESTIG MARINHA E AMBIENTAL [PT] ET) 10 September 2020 (2020-09-10) * claims 1,7 * * scheme 1; page 15 * ----- | 1-15 | TECHNICAL FIELDS SEARCHED (IPC) |
| A | GB 625 727 A (LAKELAND FOUNDATION) 4 July 1949 (1949-07-04) * page 4, line 109 - line 110 * ----- -/-- | 1-15 | |

The present search report has been drawn up for all claims

| Place of search | Date of completion of the search | Examiner |
|---|---|---|
| Munich | 22 December 2022 | Werner, Doris |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

page 3 of 4

Europäisches Patentamt
European Patent Office
Office européen des brevets

# EUROPEAN SEARCH REPORT

Application Number
EP 22 18 7606

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Munro H ET AL: "Injurious Effect of Air-saturated Water on Certain Invertebrates Chlorophyllide, the Probable Precursor of a Growth Inhibitor", Nature Publishing Group Trans. Wis . .Acad. Sci. Arts Utt, 14 August 1954 (1954-08-14), pages 312-313, XP093009216, Retrieved from the Internet: URL:https://pubmed.ncbi.nlm.nih.gov/13185298/ [retrieved on 2022-12-19] * the whole document * ----- | 1-15 | |
| | | | TECHNICAL FIELDS SEARCHED (IPC) |

The present search report has been drawn up for all claims

2

| Place of search | Date of completion of the search | Examiner |
|---|---|---|
| Munich | 22 December 2022 | Werner, Doris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 18 7606

This annex lists the patent family members relating to the patent documents cited in the above-mentioned European search report.
The members are as contained in the European Patent Office EDP file on
The European Patent Office is in no way liable for these particulars which are merely given for the purpose of information.

22-12-2022

| Patent document cited in search report | Publication date | Patent family member(s) | Publication date |
|---|---|---|---|
| KR 20200078109 A | 01-07-2020 | NONE | |
| US 2006128683 A1 | 15-06-2006 | AU 2003272116 A1 | 10-08-2004 |
| | | CA 2513133 A1 | 29-07-2004 |
| | | EP 1594875 A1 | 16-11-2005 |
| | | JP 2006514064 A | 27-04-2006 |
| | | US 2004142917 A1 | 22-07-2004 |
| | | US 2006128683 A1 | 15-06-2006 |
| | | WO 2004063200 A1 | 29-07-2004 |
| WO 2020178713 A1 | 10-09-2020 | EP 3930710 A1 | 05-01-2022 |
| | | US 2022135580 A1 | 05-05-2022 |
| | | WO 2020178713 A1 | 10-09-2020 |
| GB 625727 A | 04-07-1949 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CASSINI et al.** *Lancet Infect. Dis.,* 2019, vol. 19, 56-66
- **BLAAUW-JANSEN.** Chlorophyllide, the Probable Precursor of a Growth Inhibitor. *Nature,* vol. 174, 312-313
- **JORGENSEN, E. G.** Antibiotic Substances from Cells and Culture Solutions of Unicellular Algae with Special Reference to some Chlorophyll Derivatives. *Physiologia Plantarum,* vol. 15, 530-545
- **HANSEN, J. A.** Antibiotic Activity of the Chrysophyte Ochromonas malhamensis. *Physiologia Plantarum,* 1973, 234-238
- **AMIN ; DANNENFELSER.** In Vitro Hemolysis: Guidance for the Pharmaceutical Scientist. *Journal of Pharmaceutical Sciences,* 2006, vol. 95 (6), 1173-1176